# EUROPEAN PATENT APPLICATION

(11) **EP 2 510 922 A1**
(43) Date of publication of application: **17.10.2012**
(21) Application number: 12163907.4
(22) Date of filing: 12.04.2012
(51) Int. Cl.: A61K 9/20, A61K 31/545

(54) **Manufacturing process for tablet formulations comprising cefuroxime**

(30) Priority: 14.04.2011 TR 201103625
(71) Applicant: Sanovel Ilaç Sanayi Ve Ticaret Anonim Sirketi, 34460 Sariyer/Istanbul (TR)
(72) Inventor: Cifter, Ümit, 34460 Istanbul (TR); Türkylmaz, Ali, 34460 Istanbul (TR); Cakir, Fatih, 34460 Istanbul (TR)
(74) Representative: Sevinç, Erkan

(57) **Abstract**

The present invention provides a process for manufacturing a pharmaceutical tablet formulation comprising cefuroxime, characterized in that the relative humidity of the manufacture medium of this process is set to 20 to 40% and the compression force to be applied to the tablet is set to 10 to 20 kN.

## Description

### Field of Invention

The present invention relates to a formulation of cefuroxime or a pharmaceutically acceptable salt of cefuroxime. The present invention more particularly relates to a process for developing a stable cefuroxime formulation which has the desired features as stated below.

### Background of Invention

Cefuroxime axetil is a second-generation cephalosporin. Cefuroxime, a penicillin-like beta-lactam antibiotic, binds primarily and specifically to penicillin-binding proteins (PBPs) present on the interior of bacterial cell walls, and thus inhibits the third and final step of bacterial cell wall synthesis. The chemical designation of cefuroxime is (Z)-3-carbamoyloxymethyl-7-(2-(2-furyl)-2-methoxyiminoacetamido)-3-cephem-4-carboxylic acid, with the chemical structure illustrated below in Formula 1.

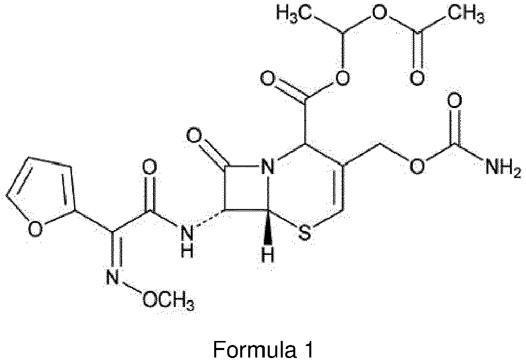

Cefuroxime is marketed under the trademark Ceftin^{®} in 125, 250, and 500 mg dosage forms. There are various patents available in the patent literature in relation to cefuroxime axetil. The patent US3974153 discloses cephalosporin antibiotics. The molecules referred to in the dependent claims of that document comprise cefuroxime (syn isomer), cefuroxime sodium (syn isomer), and the crystalline form of cefuroxime sodium (syn isomer).

The patent WO 2005018618 discloses a stable formulation of cefuroxime axetil. That formulation is protected against humidity and stabilized by means of a polyvinyl-alcohol based coating.

The patent WO9944614 discloses a stable formulation comprising cefuroxime axetil. Said formulation is protected against humidity and stabilized by means of silicon dioxide used in the formulation.

The water-solubility of the polymorph form of the active agent cefuroxime axetil is quite better as compared to its crystalline form. Its polymorph form is preferred to provide the desired bioequivalence levels in practice. External factors such as pressure, however, applied to the polymorph form during manufacture, bring about a transition into the crystalline form and result in undesired outcomes in terms of solubility and dissolution rate.

Cefuroxime axetil is very susceptible to ambient conditions. Particularly if the humidity level of a manufacture medium where such pharmaceutical forms are subjected to compression processes rises above a certain level, the degradation risk is increased undesirably since the product comprises beta-lactam groups. On the other hand, if the humidity level of the manufacture medium is at too low levels, the air absorbability of active agent particles increases as a result of these particles becoming charged with higher electrostatic charges.

Since water cannot penetrate at a sufficient rate from the surface to the interior of the tablets manufactured under these conditions, for instance, the surface becomes jellified and the disintegration time of the tablet is lengthened. This increase in the disintegration time of the tablet, in turn, causes the dissolution rate to occur at levels below those which are desired. The tablet compression force is another parameter to be taken into account besides the humidity factor in tablet manufacture. In result, the most proper relative humidity level and the most ideal compression force must be ensured for the respective production.

Based on the drawbacks mentioned above, a novelty is necessitated in the art of manufacture of cefuroxime axetil formulations having antibacterial effects.

### Object and Brief Description of Invention

The present invention provides an easily-administrable cefuroxime formulation, eliminating all aforesaid problems and brining additional advantages to the relevant prior art.

Accordingly, the main object of the present invention is to obtain a stable formulation with antibacterial activity.

Another object of the present invention is to provide such manufactured formulations with desired levels of dissolution rate and solubility, and thus with a proper bioequivalence.

A further object of the present invention is to develop a formulation not leading to flowability-related problems during manufacture.

A production process for pharmaceutical tablet formulation has been developed to carry out all objects, referred to above and to emerge from the following detailed description.

In a preferred embodiment of the present invention, said novelty has been realized by setting the relative humidity of the manufacture medium of said process to 20 to 40% and the applicable compression force to 10 to 20 kN.

In a preferred embodiment of the present invention, the relative humidity level in the manufacture medium is set to 25 to 38% and more preferably to 30 to 35%.

In another preferred embodiment of the present invention, the tablet compression force is set to 11 to 18 kN and more preferably to 12 to 16 kN.

In a further preferred embodiment of the present invention, the force applied during the pre-compression operation is set to 6 to 24 kN, preferably to 9 to 21 kN, and more preferably to 12 to 18 kN.

### Detailed Description of Invention

### Example

| **Ingredients** | **% amount (mg)** |
|---|---|
| **core** | |
| cefuroxime axetil | 60 - 70 |
| microcrystalline cellulose | 15-25 |
| croscarmellose sodium | 5-15 |
| sodium lauryl sulfate | 1 - 5 |
| hydrogenated vegetable oil, type I | 0,25 - 5 |
| colloidal silicone dioxide | 0.5 - 5 |

| **coating** | |
|---|---|
| Opadry White 02H28599 | 1 - 5 |
| ethyl alcohol* | |

| | |
|---|---|
| * removed during the film coating step. | |

The manufacture of the formulation is carried out as follows. A weighted amount of active agent cefuroxime axetil, and 85% of colloidal silicon dioxide are added into a IBC blender and stirred therein. This mixture is then sieved. To the mixture obtained are admixed microcrystalline cellulose, 75% of croscarmellose sodium, sodium lauryl sulfate, and hydrogenated vegetable oil type I (after sieving).

This uniform mixture obtained by direct mixing is passed through a compactor. The product passed through the compactor becomes ground. To this ground mixture are added colloidal silicon dioxide and croscarmellose sodium and the resulting mixture is stirred. This uniform mixture obtained is compressed in accordance with the specifications defined for each type of tablet.

The tablet cores are subjected to a film coating process with a coating solution prepared. With the process realized, a stable cefuroxime axetil formulation can be obtained which has surprisingly good solubility and dissolution rates, and thus a high bioavailability. The relative humidity of the manufacture medium in which this process is conducted is set to 20 to 40%, and the compression force to be applied to the tablet is set to 10 to 20 kN. The relative humidity of the manufacture medium can also be set to 25 to 38% and more preferably to 30 to 35%. The tablet compression force can also be set to 11 to 18 kN and more preferably to 12 to 16 kN. The force applied in the pre-compression operation can be set to 6 to 24 kN, preferably to 9 to 21 kN, and more preferably to 12 to 18 kN. According to this manufacture conditions, the tablet surface doesn't gel. In hence, obtained tablets show good solubility and stability.

This invention is a method applied to produce oral formulations comprising cefuroxime axetil, having an antibacterial effect, for preventing or treating infectious disease caused by bacteria in mammals and particularly in humans.

The pharmaceutical compositions according to the present invention may also comprise one or more pharmaceutically acceptable excipients. Such pharmaceutically acceptable excipients include, but are not restricted to fillers, binders, glidants, lubricants, disintegrants, surface active agents, etc. and the mixtures thereof.

It is further possible to use the following additional excipients in this formulation.

A filler, e.g. at least one or a mixture of lactose, starch, pregelatinized starch, microcrystalline cellulose, mannitol, dicalcium hydrogen phosphate dihydrate, calcium hydrogen phosphate anhydrate, calcium phosphate trihydrate, potassium chloride, silicium dioxide and glucose, etc.; with at least one or a mixture of dicalcium hydrogen phosphate dihydrate and/or calcium hydrogen phosphate anhydrate being preferred.

A binder, e.g. at least one or a mixture of polyvinylpyrrolidone, gelatin, sugars, glucose, natural gums, gums, synthetic celluloses, polymethacrylate, hydroxypropyl methyl cellulose, hydroxypropyl cellulose, carboxymethyl cellulose, methyl cellulose, and other cellulose derivatives, etc..

A glidant, e.g. at least one or a mixture of colloidal silicone dioxide, talk, aluminum silicate, etc...

A lubricant, e.g. at least one or a mixture of sodium stearyl fumarate, polyethylene glycol, stearic acid, metal stearates, boric acid, sodium chloride benzoate and acetate, sodium or magnesium lauryl sulfate, etc..

A surface active agent, e.g. at least one or a mixture of sodium lauryl sulfate, dioctyl sulfosuccinate, polysorbates and polyoxyethylene alkyl esters and ethers thereof, glyceryl monolaurate saponins, sorbitan laurate, sodium lauryl sulfate, magnesium lauryl sulfate, etc.. A coating agent, e.g. hydroxypropyl methyl cellulose, polyethylene glycol, polyvinylpyrrolidone, polyvinylpyrrolidone-vinyl acetate copolymer (PVP-VA), polyvinyl alcohol like polymers, and all sorts of Opadry^{™}, as well as pigments, dyes, titanium dioxide and iron oxide, talk, etc..

The present invention is hereby disclosed by referring to an exemplary embodiment hereinabove. Whilst this exemplary embodiment does not restrict the object of the present invention, the latter must be assessed under the light of the foregoing detailed description.

## Claims

1. A process for manufacturing a pharmaceutical tablet formulation comprising cefuroxime, wherein the relative humidity of the manufacture medium of this process is set to 20 to 40% and the compression force to be applied to the tablet is set to 10 to 20 kN.

2. The process for manufacturing a pharmaceutical tablet formulation comprising cefuroxime as claimed in Claim 1, wherein the relative humidity of the manufacture medium is set to 25 to 38% and more preferably to 30 to 35%.

3. The process for manufacturing a pharmaceutical tablet formulation comprising cefuroxime as claimed in any of the preceding claims, wherein the tablet compression force is set to 11 to 18 kN and more preferably 12 to 16 kN.

4. The process for manufacturing a pharmaceutical tablet formulation comprising cefuroxime as claimed in any of the preceding claims, wherein the force of a pre-compression operation applied to the formulation is set to 6 - 24 kN, preferably to 9 - 21 kN, and more preferably to 12 - 18 kN.
